# EUROPEAN PATENT SPECIFICATION

(11) **EP 3 131 910 B1**
(45) Date of publication and mention of the grant of the patent: **15.08.2018**
(21) Application number: 15720254.0
(22) Date of filing: 15.04.2015
(51) Int. Cl.: C07F 9/6521, C07F 9/6558

(54) **USE OF A 1,3,5-TRIAZIN-2-YL PHOSPHORAMIDATE COMPOUND IN THE SYNTHESIS OF SOFOSBUVIR**
VERWENDUNG EINER 1,3,5-TRIAZIN-2-YL-PHOSPHORAMIDAT-VERBIDNUNG IN DER SYNTHESE VON SOFOSBUVIR
UTILISATION D'UN COMPOSÉ PHOSPHORAMIDATE DE 1,3,5-TRIAZIN-2-YLE DANS LA SYNTHÈSE DE SOFOSBUVIR

(30) Priority: 15.04.2014 CZ 20140259
(43) Date of publication of application: 22.02.2017
(73) Proprietor: Zentiva K.S., 102 37 Praha 10 (CZ)
(72) Inventor: STEFKO, Martin, 851 01 Bratislava (SK); MAN, Stanislav, 687 32 Nezdenice (CZ); RADL, Stanislav, 250 84 Kvetnice (CZ)
(74) Representative: Jirotkova, Ivana
(86) International application number: PCT/CZ2015/000037
(87) International publication number: WO 2015/158317

(56) References cited:
- WO-A2-2011/123645

## Description

### Technical Field

The invention relates to a new type of 1,3,5-triazin-2-yl phosphoramidates of general formula **I** 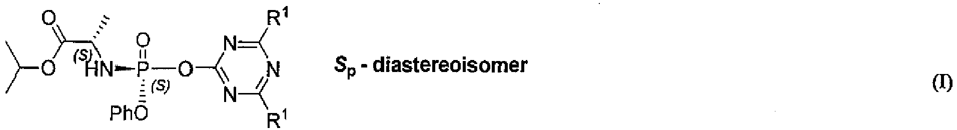 with the absolute configuration (*S*) at the phosphorus atom, an Sp diastereoisomer, wherein R¹ and R² can be independently H, a C1-C6 (un)branched alkyl, a C1-C6 (un)branched alkoxy group, a C1-C6 (un)branched alkylsulfanyl group, C1-C6 (un)branched monoalkylamino or dialkylamino group, including cyclic amino groups, e.g. pyrrolidino, piperidino or morpholino group, and their use for the production of biologically active phosphoramidate prodrugs, especially sofosbuvir of formula **II.**

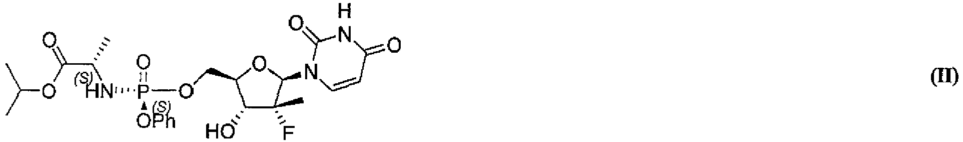

Sofosbuvir **II** is a nucleotide inhibitor of RNA polymerase, used for the treatment of hepatitis C in the form of a prodrug releasing the active antiviral agent 2'-deoxy-2'-α-fluoro-β-C-methyluridine-5'-triphosphate in the organism.

### Background Art

So far the most convenient preparation methods of sofosbuvir **II** have been based on a reaction consisting in reacting the phosphoramidate precursor (*S*p diastereoisomer **III**) with the fluorinated derivative **IV.** The reaction runs stereospecifically through the so-called S_{N}2 mechanism with inversion of the configuration at the chiral phosphorus atom. It is just this S_{N}2 mechanism that makes it possible to prepare diastereomerically pure sofosbuvir **II** by reaction of the diastereomerically pure intermediate **III** (*S*p diastereoisomer).
Literature has described a number of suitable leaving groups (LG) of the precursor **III**, including halogens (Cl, Br, I), sulfonates (e.g. mesylate, tosylate, triflate, camphor sulfonate) and aryloxy groups, preferably carrying at least one electron-attracting group (WO2008/121634, WO2010/135569, WO2011/123645, WO2012/012465, WO2014/008236). The following groups are mentioned as the most frequently used leaving groups: 4-nitrophenoxy, 2-nitrophenoxy, 2,4-dinitrophenoxy, 2-chloro-4-nitrophenoxy and pentafluorophenoxy groups (J. Org. Chem. 2011, 76, 8311). A general scheme of preparation of sofosbuvir **II** with the use of the phosphoramidate precursor of general formula **III** is shown in Scheme 1.

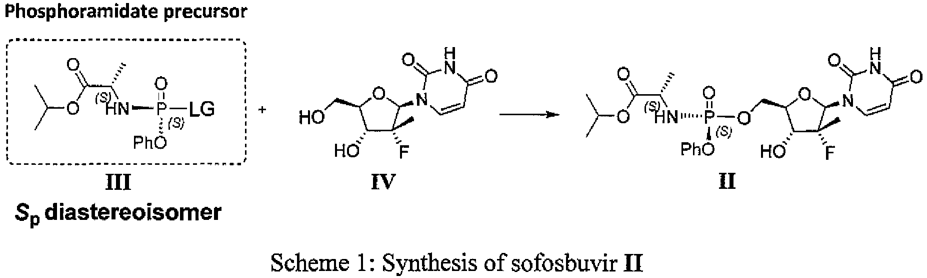

For most leaving groups the respective *S*p diastereoisomer of the phosphoramidate precursor of general formula **III** is isolated from an equimolar (1:1) mixture of the diastereoisomers by resolution (WO2010/135569) or by chromatography. In the case of phosphoramidates having an aryloxy group as the leaving group (LG) the corresponding Sp diastereoisomer can be obtained by crystallization. The patent application WO2010/135569 describes preparation of an *S*p diastereoisomer enriched mixture of 4-nitrophenoxy phosphoramidate. A crude reaction mixture containing a mixture of the *S*p and *R*p diastereoisomers (**IIIa** and **IIIb**) in the ratio of 1:1 was dissolved in a suitable solvent (CDM/IPE) and seeded with the pure *S*p diastereoisomer. The product obtained by subsequent crystallization prevalently contained the desired Sp diastereoisomer of the respective phosphoramidate **IIIa** (*S*p : *R*p 48:1; Scheme 2).

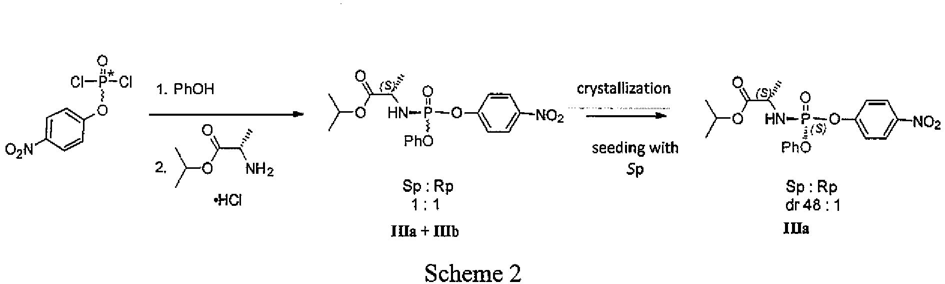

A similar procedure, this time described for a pentafluorophenoxy-substituted phosphoramidate, was described in the patent application WO2011/123645. In this document the respective optically pure *S*p diastereomer **IIIc** (de >99%) was obtained in the total yield of 37% by crystallization from a crude reaction mixture that contained both the diastereomers (**IIIc** and **IIId**) in the ratio of 1:1 (*S*p:*R*p 1:1) (Scheme 3).

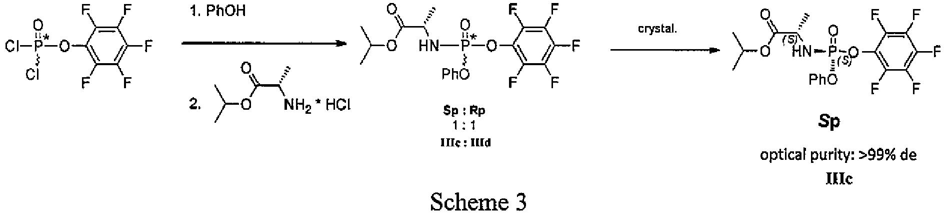

Another relatively extensive study dealt with the influence of the leaving phenoxy group of the reactivity of the phosphoramidate in a reaction with a modified nucleobase (fluoro sugar), as well as the possibility to obtain diastereomerically pure Sp phosphoramidate by means of crystallization. This is the paper already mentioned hereinabove, published in J. Org. Chem. 2011, 76, 8311, dealing especially with the phenoxy groups of the 2,4-dinitrophenol, pentafuorophenol, 2-chloro-4-nitrophenol, 2-nitropehnol, 4-nitrophenol, 2,4-dichlorophenol type. One of the possibilities of how to achieve an increase of the yield of the *S*p diastereoisomer of the phosphoramidate precursor is crystallization-induced dynamic resolution. This procedure was first described in WO2011/123645, where a pentafluorophenoxy leaving group was conveniently used, enabling to increase the total yield of the Sp diastereomer **IIIc** to 53% (Scheme 4).

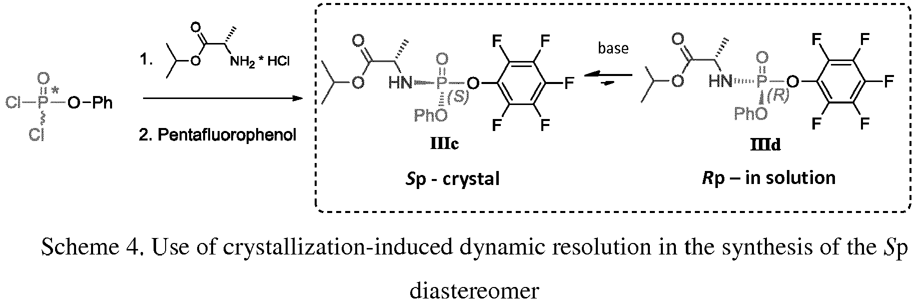

### Disclosure of Invention

The invention provides a new improved process for producing biologically active phosphoramidate prodrugs, especially sofosbuvir of formula **II.** This new synthetic strategy uses the good availability of diastereoisomerically pure phosphoramidate precursors of type **I** as the key intermediates in the industrial production of sofosbuvir of formula **II** (Scheme 5).

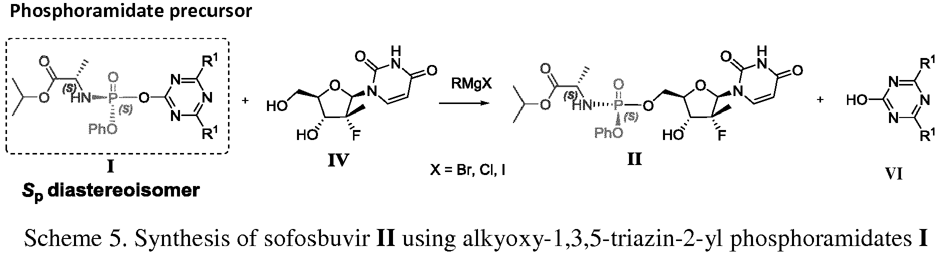

This invention is based on the surprising observation that replacement of the aryloxy group in formula **III** with a suitably substituted 1,3,5-triazin-1-yloxy group of the structure of formula **I,** wherein R¹ and R² can independently be H, a C1-C6 unbranched or branched alkyl, C1-C6 unbranched or branched alkoxy group, C1-C6 unbranched or branched alkylsulfanyl group, C1-C6 unbranched or branched monoalkylamino or dialkylamino group, pyrrolidino group, piperidino group or morpholino group, as well as their stereoisomers, salts, solvates, hydrates and crystalline and amorphous forms, can lead to achievement of both an improvement of the regioselective substitution (problem outlined in the document WO2010/135569) and an increase of the yields and simplified purification of the final products. Besides the reactivity, a suitable selection of the R¹, R² groups can significantly influence solubility of 1,3,5-triazines of formula **VI** in water and organic solvents, which is just a way isolation and purification of the final products can be facilitated. 1,3,5-Triazines of formula **VI** substituted with the short C1-C2 alkyl groups - methoxy, ethoxy or dimethylamino - or carrying polar groups, e.g. *N*-methylpiperazino or trialkylammonium, are relatively well soluble in water and after the completion of the reaction they can be removed from the reaction mixtures by mere extraction from the organic solvent into water without using of any other agents.
The other aspects of the present invention provide the following compounds:
a) The compound having the structure of formula **Ia** 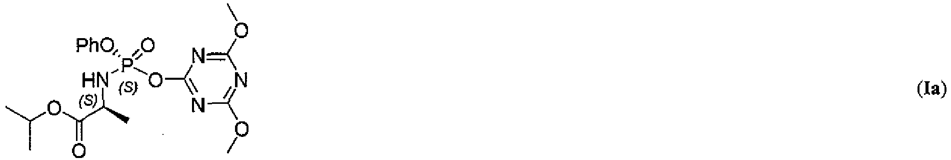 and whose name is isopropyl 2-(*S*)-[(*S*)-(4,6-dimethoxy-1,3,5-triazin-2-oxy)-phenoxy-phosphorylamino]-propionate;
b) The compound having the structure of formula **Ib** 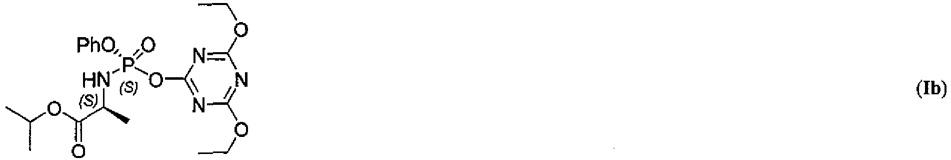 and whose name is isopropyl 2-(*S*)-[(*S*)-(4,6-diethoxy-1,3,5-triazin-2-oxy)-phenoxy-phosphorylamino]-propionate;
c) The compound having the structure of formula **Ic** 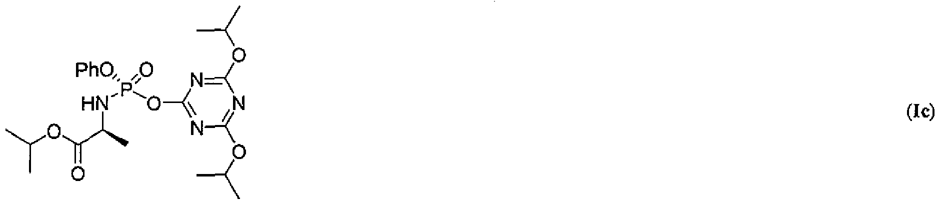 and whose name is isopropyl 2-(*S*)-[(*S*)-(4,6-diisopropoxy-1,3,5-triazin-2-oxy)-phenoxy-phosphorylamino]-propionate;
d) The compound having the structure of formula **Id** 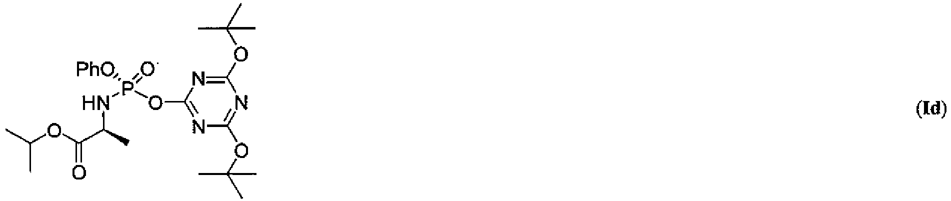 and whose name is isopropyl 2-(*S*)-[(*S*)-(4,6-di-tert-butoxy-1,3,5-triazin-2-oxy)-phenoxy-phosphorylamino]-propionate.

A suitably selected arrangement of the experiment - suitable substituents - then makes it possible in the case of aqueous processing of the reaction mixture to transfer the secondary product of the reaction - the hydroxytriazine of formula **VI** into the aqueous phase while the product of formula **II** remains in the organic layer, which represents the input raw material of the next technological stage - crystallization (Scheme 5).

However, if 1,3,5-triazines of formula **VI** are substituted with substituents with longer (C3-C6) or branched alkyl chains (e.g. propoxy, isopropoxy, allyloxy, butoxy, isobutoxy, *tert-*butoxy, diethylamino), they are strongly lipophilic and therefore they dissolve well in organic solvents. But if during the preparation of sofosbuvir of formula **II** such a lipophilic triazine of formula **VI** is produced, then, on the other hand, it does not pass or only partly passes into the aqueous phase during the processing and washing. The above-mentioned lipophilic triazines of formula **VI** are, unlike the water-soluble ones of formula **VI,** easy to remove from the compound of formula **II** by crystallization thanks to their high solubility in organic solvents. This is just another one of the surprising advantages of the hydroxytriazines of formula **VI.** In fact, the hydroxytriazines of formula **VI,** compared to pentafluorophenol as the leaving group, provide a purer final product **in both the cases** (using both a lipophilic and hydrophilic triazine) than with the use of the above-mentioned pentafluorophenol. This is because pentafluorophenol still remains in the product no matter whether purification by crystallization or by extraction is used. The most convenient R¹, R² groups of substituted hydroxytriazines of formula **VI** are alkoxy groups, preferably wherein especially R¹ = R² is methoxy, ethoxy, propoxy, allyloxy, isopropoxy or *tert*-butoxy.
According to the procedure described in this invention the triazines of formula **VI** were prepared in high yields from the respective 2,4,6-trialkoxy-1,3,5-triazines of formula **VII,** obtained by reaction of the respective alcoholates with cyanuric chloride. Preparation of formula **VI** from cyanuric chloride can be carried out not only as a two-step process, but these substances can be advantageously prepared by a *"one-pot"* process. Highly selective substitution of just one alkoxy group with the hydroxy group can be carried out by treatment with hydroxides in the respective alcohol, *tert*-butanol or in water (Scheme 6).

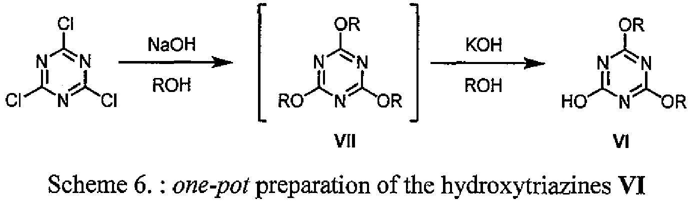

The key benefit of the present method is a low cost of the starting raw materials, which is one of the key criteria for industrial utilization of this procedure. Other undisputed benefits include very easy handling of the triazines of formula **VI,** which are, compared to phenolic substrates, non-volatile, non-irritant and solid crystalline substances without any odor. In terms of the technological process and of their use for the production of sofosbuvir of formula **II** the respective triazines of formula **VI** represent a side product of the reaction (Scheme 5). Thanks to their unique physical-chemical characteristics they can be easily separated from the reaction mixture and subsequently easily decomposed by the action of mineral acids onto the non-toxic cyanuric acid. In addition, these triazines can be safely incinerated without any unnecessary risks. This method and their use represent a "green" alternative to the original methods described in the literature, using phenolic derivatives.

The synthesis of diastereoisomerically pure phosphoramidates of formula I is based on reaction of (*S*)-isopropyl 2-aminopropanoate (*L*-alanine isopropyl ester) with phenyl dichlorophosphate in the presence of a suitable base in suitable solvents, and subsequent reaction of the produced intermediate of formula **V** with a suitable triazine of formula **VI** (Scheme 7). Bases suitable for this reaction step include especially bases from the series of amines, such as triethylamine (Et₃N), N,N-diisopropylethylamine (DIPEA), 1,8-diazabicyclo[5.4.0]undec-7-ene (DBU), 1,4-diazabicyclo[2.2.2]octane (DABCO), 1-methylmorpholine, 1-ethylpiperidine, further carbonates and hydrogen carbonates such as Na₂CO₃, K₂CO₃, Cs₂CO₃, NaHCO₃, KHCO₃, or acetates NaOAc and KOAc in a wide range of solvents and their mixtures. In a preferred embodiment triethylamine or DIPEA are used for this reaction step. Dichloromethane, chloroform, tetrahydrofuran, 2-methyltetrahydrofuran, cyclopentyl methyl ether, dimethoxy ethane, *tert-butyl* methyl ether (MTBE), toluene, trifluorotoluene (TFT) have proved to be suitable solvents for this reaction. In a preferred embodiment this reaction is carried out in dichloromethane, tetrahydrofuran, 2-methyltetrahydrofuran, cyclopentyl methyl ether. The above-mentioned reaction step is conducted at temperatures of from -70°C to 20°C, preferably in the range from -40°C to 0°C. In the preparation of the compound of formula **I**, the intermediate of formula **V** does not need to be isolated and it is conveniently generated *in situ.* The subsequent reaction of the compound of formula **V** with a suitably selected triazine of formula **VI** usually results in obtaining an equimolar mixture of the diastereoisomers Sₚ:Rₚ of formula **IX** in the ratio of 1:1. However, depending on the selected conditions other ratios of the diastereoisomers Sₚ:Rₚ of formula **IX** may also be obtained. This reaction is conducted at temperatures of -10 to 10°C, preferably at 0°C.
Direct separation of the target Sp diastereoisomer of formula **I** by means of crystallization is The main advantage of the above-mentioned process. Using this method the Sp-diastereoisomer of the phosphoramidate precursor of formula **I** can be obtained in good total yields (>50%) and an excellent optical purity (>99% de) from the crude reaction mixture (Scheme 7). The most convenient R¹, R² groups of the compounds of formula **I** are alkoxy groups, preferably wherein especially R¹ = R² is the methoxy, ethoxy, propoxy, allyloxy, isopropoxy or *tert*-butoxy group.

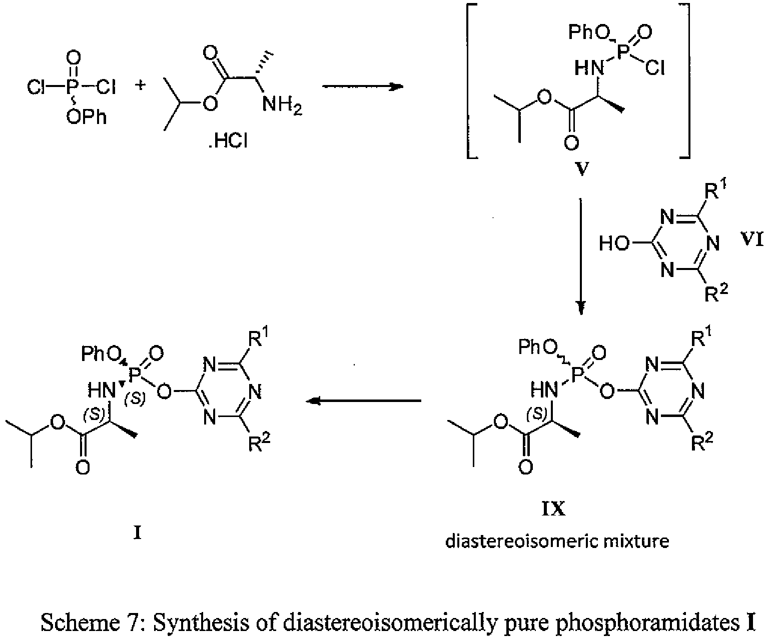

In addition, it has been surprisingly found out that the triazine phosphoramidates are also subject to crystallization-induced dynamic resolution, such that the yield of directly obtained product of formula **I** can still be increased this way. Pure *S*p diastereomer of the precursor of formula **I** can be prepared from the *R*p diastereomer, starting from pure *R*p isomer or any mixture of the *S*p : *R*p diastereoisomers of formula **IX,** preferably from a mixture containing a higher amount of the *R*p isomer. By the treatment of *R*p or any *S*p : *R*p mixture with bases, such as Et₃N, DIPEA, DABCO, DBU, imidiazole, 1,2-bis(dimethylamino)ethane, 1,2-bis(diethylamino)ethane, 1,3-bis(dimethylamino)propane, 1,3 -bis(diethylamino)propane, changes of the absolute configuration at the P atom can be achieved in suitable solvents; Et₃N or DIPEA being used as the base in preferred embodiments. Suitable solvents for crystallization-induced dynamic resolution include especially ethyl acetate (EtOAc), isopropyl acetate (*i*-PrOAc), 2-methyltetrahydrofuran, tetrahydrofuran (THF), dioxane, dimethoxyethane, diethoxyethane, *tert*-butyl methyl ether (MTBE), cyclopentyl methyl ether, diethyl ether, toluene, methyl ethyl ketone (MEK), acetone; further non-polar solvents, especially hydrocarbons in general - pentane, hexane, heptane, cyclohexane, decalin, methylcyclohexane, 2-methylbutane, 2-methylpentane, triethylmethane, isooctane; aromatic solvents - toluene, xylene, mesitylene, benzene, trifluorotoluene (TFT); and combinations of the above-mentioned solvents. EtOAc, *i*-PrOAc, 2-methyltetrahydrofuran (2-MeTHF), tetrahydrofuran, hexane, heptane, cyclohexane and their mixtures are used in preferred embodiments.
Conditions for the preparation of pure *S*p-diastereoisomers of formula **I** in accordance with this invention are simple and lead to high yield of chirally very pure products. This invention also describes a convenient embodiment wherein crystallization-induced dynamic resolution was conducted without separation of pure *S*p diastereoisomers of formula **I** from the reaction mixture and these *S*p diastereoisomers of formula **I** were only isolated afterwards. The new triazine phosphoramidates of general formula **I** described in this patent are stable, sufficiently reactive, selective and generally usable for industrial production of biologically active phosphoramidate prodrugs, especially of sofosbuvir of formula **II.** The most convenient R¹, R² groups of the compounds of formula **I** are alkoxy groups, preferably wherein especially R¹ = R² is the methoxy, ethoxy, propoxy, allyloxy, isopropoxy or *tert*-butoxy group.
This invention also comprises the use of the new triazine phosphoramidates of general formula **I** in the production of sofosbuvir of formula **II.** In this procedure the intermediate of formula **IV** can be activated by treatment with virtually any Grignard reagent (Scheme 5). Generally, any primary, secondary and tertiary magnesium chlorides, bromides and iodides can be used, such as *tert-butyl* magnesium chloride, benzylmagnesium chloride, cyclohexylmagnesium chloride, cyclohexylmagnesium bromide, heptylmagnesium chloride, octylmagnesium chloride. In preferred embodiments heptylmagnesium chloride and *tert-butyl* magnesium chloride are used. In an embodiment in accordance with this invention the Grignard reagent : intermediate of formula **IV** are used in ratios of 1.1:1 to 2.9:1 molar equivalents, preferably in ratios of 1.8:1 to 2.2:1. Suitable solvents or their mixtures include especially THF, 2-MeTHF, dioxane, MTBE, cyclopentyl methyl ether, Et₂O, dimethoxyethane, diethoxyethane, dimethoxypropane, diethoxypropane; preferably THF, 2-MeTHF and cyclopentyl methyl ether are used. It has also been surprisingly found out that to achieve high conversion and regioselectivity the input intermediate of formula **IV** does not need to be completely dissolved in the solvent or solvent mixture used before addition of the Grignard reagent. From the industrial point of view, using such Grignard reagents that release higher boiling hydrocarbons during the reaction (e.g. toluene, cyclohexane, heptane, octane) may be of a great practical and safety advantage. Triazine phosphoramidates of formula **I** react very well with the intermediate of formula **IV** after activation by the Grignard reagent at the temperatures of -20 to 20°C, preferably at -10 to 10°C. According to the production process presented in this patent the triazine phosphoramidates of formula **I** react with the intermediate of formula **IV** in molar ratios of 10:1 to 1:10, preferably 2:1 to 1:2. This process and the use of the triazine phosphoramidates of formula **I** in the production of sofosbuvir of formula **II** provide outstanding yields with excellent chemical and optical purity of the sofosbuvir obtained. These characteristics clearly predetermine the entire process for industrial production of sofosbuvir of formula **II** in a quality that can be directly used for the production of the final dosage form.
The invention is clarified in a more detailed way using the working examples below. These examples, which illustrate the improvement of the procedure in accordance with the invention, only have an illustrative character and do not restrict the scope of the invention in any respect.

### Examples

The reactions were routinely monitored with the use of Agilent 1100 HPLC apparatus with a UV (PDA) detectors equipped with an Ascentis Express C18 column (2.7 µm) (100 x 4.6 mm) at the flow rate of 1 ml/min, mobile phase **A:** phosphate buffer (pH ~ 3) and phase **B:** acetonitrile (0 - 2 min 10% MeCN, 2 - 18 min gradient 10 -> 90% MeCN). The ¹H, ¹³C and ³¹P NMR spectra were recorded using NMR Bruker Avance 250 or 500 MHz spectrometers.

### 2,4,6-Trialkoxy-1,3,5-triazines

### Example 1

### 2,4,6-Trimethoxy-1,3,5-triazine of formula VIIa

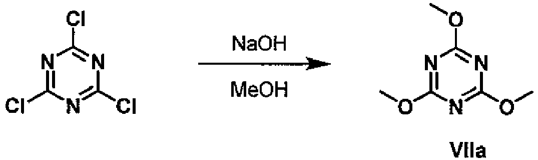

Cyanuric chloride (92 g, 0.5mol) was added to a mixture of NaOH (60 g, 1.5 mol) and MeOH (500 ml) in small portions at a temperature of 0 - 5°C. After addition of all the cyanuric chloride the mixture was agitated at 20°C for 2 h. The solvent was evaporated *in vacuo* and the distillation residue was suspended in H₂O (500 ml). The product was aspirated, washed with H₂O (2 x 40 ml) and dried at the room temperature and pressure. 2,4,6-Trimethoxy-1,3,5-triazine of formula **VIIa** was obtained as a solid colorless substance (79 g, 92%). ¹H-NMR (250 MHz, DMSO-d₆): 3.92 (s, 9H; OC*H*₃) ppm. ¹³C-NMR (63 MHz, DMSO-d₆): 173.1, 55.1 ppm. HPLC (200 nm): *R*ₜ = 6.072 min.

### Example 2

### 2,4,6-Triethoxy-1,3,5-triazine of formula VIIb

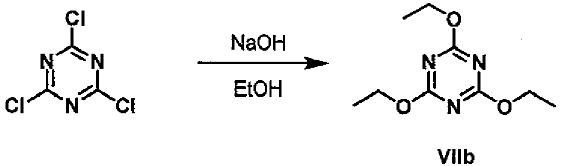

Using the procedure described in Example 1, 2,4,6-triethoxy-1,3,5-triazine of formula **VIIb** was obtained as slowly crystallizing colorless oil in the 93% yield (99 g, 93%). ¹H-NMR (250 MHz, DMSO-d₆): 4.34 (q, *J* = 7.1, 6 H; OC*H*₂), 1.30 (t, *J* = 6.2, 18 H; C*H*₃) ppm. ¹³C-NMR (63 MHz, DMSO-d₆): 172.5, 63.6, 14.0 ppm. HPLC (200 nm): *R*ₜ = 11.383 min.

### Example 3

### 2,4,6-Triisopropoxy-1,3,5-triazine of formula VIIc

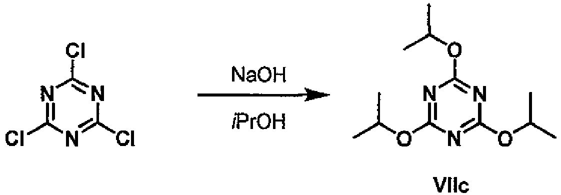

Using the procedure described in Example 1, 2,4,6-triisopropoxy-1,3,5-triazine of formula **VIIc** was obtained as a solid colorless substance in the 69% yield. ¹H-NMR (250 MHz, DMSO-d₆): 5.35 (sept, *J* = 6.2, 3 H; OC*H*), 1.37 (d, *J* = 6.2, 18 H; C*H*₃) ppm. ¹³C-NMR (63 MHz, DMSO-d₆): 172.6, 71.3, 21.8 ppm. HPLC (200 nm): *R*ₜ = 14.625 min.

### Example 4

### 2,4,6-Tri(tert-butoxy) -1,3,5-triazine of formula VIId

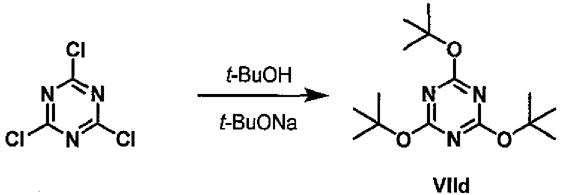

A solution of *t*BuOK in THF (1 M, 32 ml) was added dropwise to a solution of cyanuric chloride (1.84 g, 0.01 mol) in THF (7 ml) at 20°C in the course of 90 min. The reaction mixture was agitated at 20°C for 4 h and subsequently concentrated *in vacuo.* The evaporation product was suspended in H₂O (20 ml). The solid product was isolated by filtration, washed with water (3 x 10 ml) and then dried at the pressure of 0.1 kPa and temperature of 20°C. The triazine of formula **VIId** was obtained as a colorless solid substance in the 87% (2.58 g) yield. ¹H-NMR (250 MHz, DMSO-d₆): 1.53 (s, 27 H; C*H*₃) ppm. ¹³C-NMR (63 MHz, DMSO-d₆): 171.2, 81.8, 27.9 ppm. HPLC (200 nm): *R*ₜ = 16.815 min.

### 2-Hydroxy-4,6-dialkoxy-triazines

### Example 5

### 4,6-Dimethoxy-1,3,5-triazin-2-ol of formula VIa

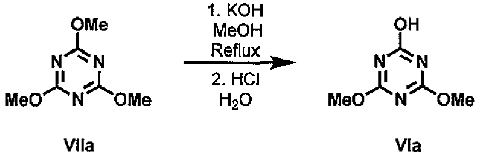

A mixture of 2,4,6-trimethoxy-1,3,5-triazine of formula **VIIa** (34 g, 0.2 mol) and KOH (14 g, 0.25 mol) in MeOH (150 ml) was heated up to boiling for 20 h. After cooling down to the room temperature the solvent was evaporated *in vacuo* and the distillation residue was put in water (50 ml). The mixture was acidified with AcOH to pH ∼6 at a temperature below 15°C. The separated product was aspirated, washed with H₂O (2 x 35 ml) and dried at the room temperature and pressure. 2-Hydroxy-4,6-dimethoxy-1,3,5-triazine of formula **VIa** was obtained as a colorless crystalline substance (27 g, 87%). ¹H-NMR (250 MHz, DMSO-d₆): 12.42 (bs, 1 H), 3.86 (s, *6* H; OC*H*₃) ppm. ¹³C-NMR (63 MHz, DMSO-d₆): 168.1, 157.3, 55.2 ppm. HPLC (200 nm): *R*ₜ = 1.475 min.

### Example 6

### 4,6-Diethoxy-1,3,5-triazin-2-ol of formula VIb

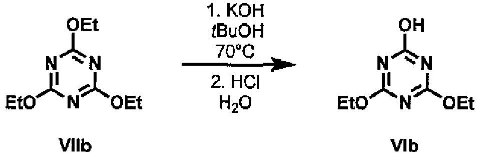

A mixture of 2,4,6-triethoxy-1,3,5-triazine of formula **VIIb** (21 g, 0.1 mol) and KOH (7.7 g, 0.137 mol) in *t*BuOH (100 ml) was heated up to 70°C for 2 h. After cooling down to the room temperature the solvent was evaporated *in vacuo* and the distillation residue was put in water (100 ml). The mixture was acidified with concentrated aqueous hydrochloric acid to pH ∼3 at a temperature below 15°C. The separated product was aspirated, washed with H₂O (2 x 25 ml) and dried at the room temperature and pressure. 2-Hydroxy-4,6-diethoxy-1,3,5-triazine of formula **VIb** was obtained as a colorless crystalline substance (15.3 g, 84%). ¹H-NMR (250 MHz, DMSO-d₆): 12.31 (bs, 1 H), 4.31 (q, *J* = 7.1, 4 H; C*H*₂), 1.27 (t, *J* = 7.1, 6 H, C*H*₃) ppm. ¹³C-NMR (63 MHz, DMSO-d₆): 168 (bs), 157.1, 64.1, 14.0 ppm. HPLC (200 nm): *R*ₜ = 5.154 min.

### Example 7

### 4,6-Diisopopoxy-1,3,5-triazin-2-ol of formula VIc

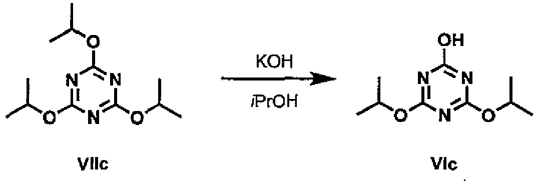

A mixture of 2,4,6-triisopropoxy-1,3,5-triazine of formula **VIIc** (88 g, 0.345 mol) and KOH (24 g, 0.43 mol) in *i*PrOH (260 ml) was heated up to boiling for 2 h. After cooling down to the room temperature the solvent was evaporated *in vacuo* and the distillation residue was put in water (260 ml). The mixture was acidified with concentrated aqueous hydrochloric acid to pH ∼3 at a temperature below 15°C. The separated product was aspirated, washed with H₂O (3 x 150 ml) and dried at the room temperature and pressure. 2-Hydroxy-4,6-diisopropoxy-1,3,5-triazine of formula **VIc** was obtained as a colorless crystalline substance (64 g, 87%). ¹H-NMR (250 MHz, DMSO-d₆): 12.19 (bs, 1H), 5.18 (sept, *J* =6.2, 2 H; OC*H*), 1.27 (d, *J* = 6.2,12 H; C*H*₃) ppm. ¹³C-NMR (63 MHz, DMSO-d₆): 167 (bs), 157.1, 71.7,21.4 ppm. HPLC (200 nm): *R*ₜ = 7.883 min.

### Example 8

### 4,6-Di(tert-butoxy)-1,3,5-triazin-2-ol of formula VId

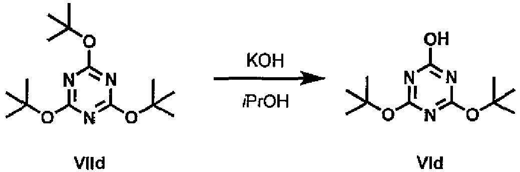

A mixture of 2,4,6-tri(*tert*-butoxy)-1,3,5-triazine of formula **VIId** (0.6 g, 2 mmol) and KOH (0.14 g, 2.5 mmol) in *t*BuOH (260 ml) was heated up to 70°C for 5 h. After cooling down to 20°C the mixture was concentrated *in vacuo.* The evaporation product was mixed with H₂O (5 ml) and acidified to pH ∼ 6 with an aqueous solution of acetic acid (10%). The separated solid product was aspirated, washed with water (3 x 5 ml) and dried *in vacuo* (0.1 kPa) at 20°C. The product of formula **VId** was obtained as a light yellow solid substance in the yield of 55% (0.27 g). ¹H-NMR (250 MHz, DMSO-d₆): 11.99 (bs, 1H), 1.54 (s, 18 H; C*H*₃) ppm. ¹³C-NMR (63 MHz, DMSO-d₆): 167 (bs), 156.5, 84 (bs), 27.8 ppm.

### Phosphorus intermediates

### Example 9

### Isopropyl 2-(S)-[(S)-(4,6-dimethoxy-1,3,5-triazm-2-oxy)-phenoxy-phosphorylamino]propionate of formula Ia

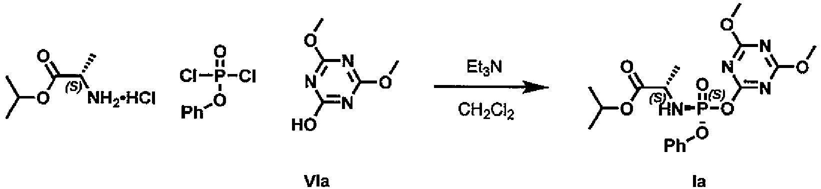

A solution of Et₃N (55 g, 0.55 mol) in CH₂Cl₂ (300 ml) was added dropwise to a mixture of isopropyl alanine.HCl (44 g, 0.26 mol) and phenyl dichlorophosphate (57 g, 0.27 mol) in CH₂Cl₂ (300 ml) at a temperature of -50 to -60°C. After 30 minutes' agitation at -50 to -60°C the reaction mixture was heated up to -5°C in 2 h. A solution of 2-hydroxy-4,6-dimethoxy-1,3,5-triazin-2-ol of formula **VIa** (41 g, 0.26 mol) and Et₃N (29 g, 0.29 mol) in CH₂Cl₂ (160 ml) was added to the reaction mixture dropwise at a temperature of -10 to 0°C. The reaction mixture was agitated at a temperature of -10 to 0°C for 16 h. The reaction mixture was washed with H₂O (4 x 300 ml). The solvent was evaporated *in vacuo* and the product was crystallized from a mixture of diethyl ether and cyclohexane. Isopropyl 2-(*S*)-[(*S*)-(4,6-dimethoxy-1,3,5-triazin-2-oxy)-phenoxy-phosphorylamino] propionate (**Ia**) was obtained as a light yellow crystalline substance (21 g, 19%). ¹H-NMR (500 MHz, DMSO-d₆): 7.41 (t, *J* = 7.9, 2 H; Ph), 7.27 (d, *J* = 7.3, 1 H; Ph), 7.23 (t, *J* = 7.4, 1 H; Ph), 6.55 (dd, *J* = 13.0, *J* = 10.1, 1 H; NH), 4.81 (sept, *J* = 6.3, 1 H; OC*H*), 4.11 - 4.00 (m, 1 H; NH-C*H*), 3.96 (s, 6 H; OC*H*₃), 1.24 (d, *J* = 7.1, 3 H; NHCHC*H*₃), 1.14 (d, *J* = 6.3, 3 H; OCHCₐ*H*₃), 1.11 (d, *J* = 6.3, 3 H; OCHC_{b}*H*₃) ppm. ³¹P-NMR (101 MHz, DMSO-d₆): -3.24 ppm. ¹³C-NMR (126 MHz, DMSO-d₆): 173.3, 172.2 (d, *J* = 5,3), 168.3 (d, *J* = 2,5), 150.0 (d, *J* = 6,1), 129.8, 125.1, 120.2 (d, *J* = 4.9), 72.0, 68.0, 50.0 (d, *J* = 1.1), 21.26, 21.25, 19.9 (d, *J* = 6.6) ppm. HPLC (210 nm): *R*ₜ = 12.295 min.

### Example 10

### Isopropyl 2-(S)-[(S)-(4,6-diethoxy-1,3,5-triazin-2-oxy)-phenoxy-phosphorylamino]propionate of formula Ib

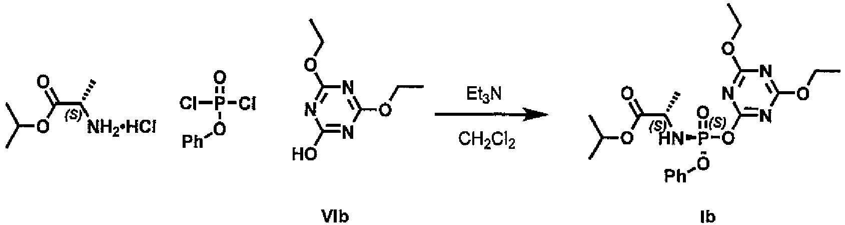

Using the procedure described in Example 9, isopropyl 2-(*S*)-[(*S*)-(4,6-diethoxy-1,3,5-triazin-2-oxy)-phenoxy-phosphorylamino]propionate of formula **Ib** was obtained as a colorless crystalline substance in the 38% yield.
¹H-NMR (500 MHz, DMSO-d₆): 7.41(t, *J* = 7.9, 2 H; Ph), 7.26 (d, *J* = 8.6, 1 H; Ph), 7.23 (d, *J* = 7.4, 1 H; Ph), 6.54 (dd, *J* = 13.2, *J* = 10.0, 1 H; NH), 4.81 (sept, *J* = 6.4, 1 H; OC*H*), 4.39 (q, *J* = 7.1; 4 H, OC*H*₂), 4.07 - 3.97 (m, 1 H; NH-C*H*), 1.32 (t, *J* = 7.1, 6 H; 2 x OCH₂C*H*₃), 1.23 (d, *J* = 7.1, 3 H; NHCHC*H*₃), 1.13 (d, *J* = 6.3, 3 H; OCHCₐ*H*₃), 1.11 (d, *J* = 6.2, 3 H; OCHC_{b}*H*₃) ppm. ³¹P-NMR (101 MHz, DMSO-d₆): -3.34 ppm. ¹³C-NMR (126 MHz, DMSO-d₆): 172.7, 172.2 (d, *J* = 5.4), 168.2 (d, *J* = 2,3), 150.0 (d, *J* = 6,1), 129.8, 125.1, 120.2 (d, *J* = 4.9), 68.1, 64.4, 50.1 (d, *J* = 1.5), 21.31, 21.26, 19.9 (d, *J* = 6.7), 13.9 ppm. HPLC (210 nm): *R*ₜ = 13.954 min.

### Example 11

### Isopropyl 2-(S)-[(S)-(4,6-diisopropoxy-1,3,5-triazin-2-oxy)-phenoxy-phosphorylamino]propionate of formula Ic

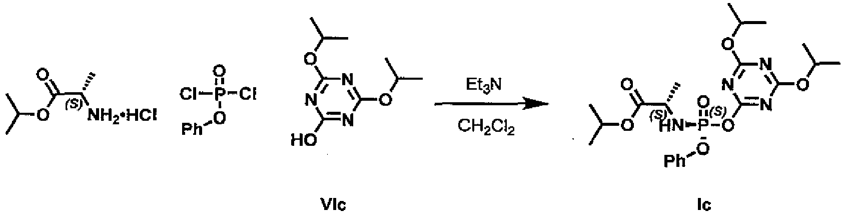

Using the procedure described in Example 9, isopropyl 2-(*S*)-[(*S*)-(4,6-diisopropoxy-1,3,5-triazin-2-oxy)-phenoxy-phosphorylamino]propionate of formula **Ic** was obtained as a colorless crystalline substance in the 47% yield.
¹H-NMR (500 MHz, DMSO-d₆): 7.41 (t, *J* = 7.9, 2 H; Ph), 7.25 (d, *J* = 8.6, 1 H; Ph), 7.22 (t, *J* = 7.5, 1 H; Ph), 6.52 (dd, *J* = 13.4, *J* = 10.0, 1 H; NH), 5.21 (sept, *J* = 6.2, 1 H; N=C-OC*H*), 4.82 (sept, *J* = 6.3, 1 H; OC-OC*H*), 4.05 - 3.95 (m, 1 H; NH-C*H*), 1.312 (d, *J* = 6.1, 6 H; N=C-OCHCₐ*H*₃), 1.310 (d, *J* = 6.3, 6 H; N=C-OCHC_{b}*H*₃), 1.24 (d, *J* = 7.1, 3 H; NHCHC*H*₃), 1.13 (d, *J* = 6.2, 3 H; O=C-OCHCₐ*H*₃), 1.10 (d, *J* = 6.2, 3 H; O=C-OCHC_{b}*H*₃) ppm. ³¹P-NMR (101 MHz, DMSO-d₆): -3.45 ppm. ¹³C-NMR (126 MHz, DMSO-d₆): 172.3, 172.1 (d, *J* = 5.6), 168.3 (d, *J* = 2,5), 150.0 (d, *J* = 6.1), 129.8, 125.1, 120.2 (d, *J* = 4.9), 72.0, 68.2, 50.0 (d, *J* = 1.1), 21.38, 21.36, 21.34, 21.24, 19.9 (d, *J* = 6.6) ppm. HPLC (210 nm): *R*ₜ = 15.272 min.

### Example 12

### Isopropyl 2-(S)-[(S)-(4,6-diisopropoxy-1,3,5-triazin-2-oxy)-phenoxy-phosphorylamino]propionate of formula Ic

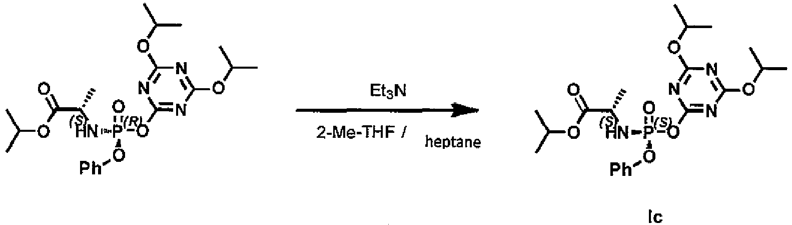

Isopropyl 2-(*S*)-[(*R*)-(4,6-diisopropoxy-1,3,5-triazin-2-oxy)-phenoxy-phosphorylamino]-propionate (1 g) (*R*p : *S*p > 80 : 20) was dissolved in 2-methyltetrahydrofuran (1 ml). Heptane (5 ml) and DIPEA (0.2 ml) were added to the solution. The mixture was agitated at the room temperature for 4 days. The separated product was aspirated and washed with a mixture of 2-methyltetrahydrofuran / heptane. Isopropyl 2-(S)-[(S)-(4,6-diisopropoxy-1,3,5-triazin-2-oxy)-phenoxy-phosphorylamino]propionate of formula **Ic** was obtained as a colorless crystalline substance in the yield of 45% (0.454 g).

### Example 13

### Isopropyl 2-(S)-[(S)-(4,6-diisopropoxy-1,3,5-triazin-2-oxy)-phenoxy-phosphorylamino]propionate of formula Ic

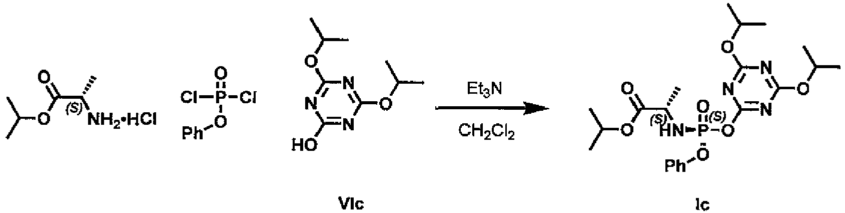

Using the procedure described in Example 9, isopropyl 2-(*S*)-[(*S*)-(4,6-diisopropoxy-1,3,5-triazin-2-oxy)-phenoxy-phosphorylamino]propionate of formula **Ic** was obtained as a colorless crystalline substance in the 46% (57 g) yield. Mother liquors, containing a *S*p : *R*p ∼13 : 87 mixture, were concentrated *in vacuo* and dissolved in EtOAc (51 ml) again. Cyclohexane (255 ml) and then Et₃N (10 ml) were slowly added to the solution. The mixture was agitated at the room temperature for 2 days. The separated product was aspirated and washed with a mixture of EtOAc / cyclohexane and dried *in vacuo* with the yield of 27% (34 g) of the second fraction of the crystalline isopropyl 2-(*S*)-[(*S*)-(4,6-diisopropoxy-1,3,5-triazin-2-oxy)-phenoxy-phosphorylamino]propionate of formula **Ic.**

### Example 14

### Isopropyl 2-(S)-{(S)-[4,6-di(tert-butoxy)-1,3,5-triazin-2-oxy]-phenoxy-phosphorylamino}propionate of formula Id

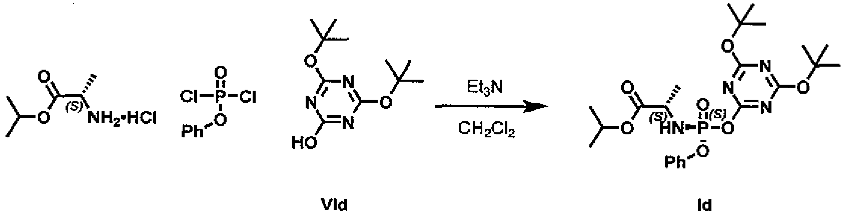

Using the procedure described in Example 9 isopropyl 2-(S)-{(S)-[4,6-di(*tert*-butoxy)-1,3,5-triazin-2-oxy]-phenoxy-phosphorylamino}propionate (**Id**) was obtained as a colorless crystalline substance in the 14% yield. ¹H-NMR (500 MHz, DMSO-d₆): 7.40 (t, *J* = 7.9, 2 H; Ph), 7.28 (d, *J* = 7.3, 1 H; Ph), 7.23 (t, *J* = 7.4, 1 H; Ph), 6.54 (dd, *J* = 13.0, *J* = 10.1, 1 H; NH), 4.81 (sept, *J* = 6.3, 1 H; OC*H*), 4.11 - 4.00 (m, 1 H; NH-C*H*), 1.53 (s, 18 H; C(C*H*₃)₃), 1.24 (d, *J* = 7.1, 3 H; NHCHC*H*₃), 1.14 (d, *J* = 6.3, 3 H; OCHCₐ*H*₃), 1.12 (d, *J* = 6.3, 3 H; OCHC_{b}*H*₃) ppm. ³¹P-NMR (101 MHz, DMSO-d₆): -3.48 ppm. ¹³C-NMR (126 MHz, DMSO-d₆): 172.9, 172.2 (d, *J* = 5.3), 168.2 (d, *J* = 2.5), 150.5 (d, *J* = 6.1), 129.8, 125.1, 120.2, (d, *J* = 4.9), 81.8, 68.0, 50.3, (d, *J* = 1.4), 27.8, 21.30, 21.25, 19.9 (d, *J* = 6.7) ppm.

### Target compounds

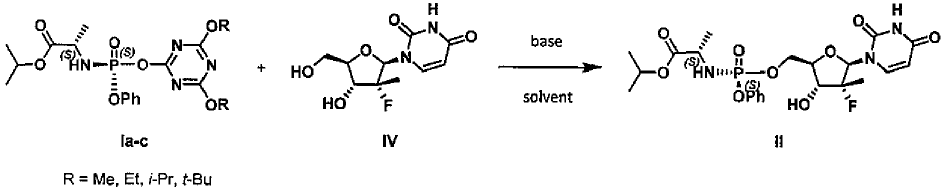

### Example 15

### Sofosbuvir of formula II

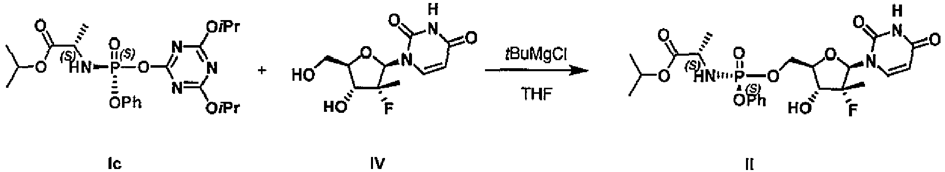

A solution of *tert*-BuMgCl (17.35 ml, 17.35 mmol, 2.1 equiv. 1M solution in THF) was added dropwise to a solution of the compound of formula **IV** (2.15 g, 8.26 mmol) in THF (66 ml) at -15°C during 1 hour. The reaction mixture was agitated at -15°C for 1h, then heated up to -5°C during 30 min and agitated for another 2h. Then, a solution of 2-(*S*)-[(*S*)-(4,6-diisopropoxy-1,3,5-triazin-2-oxy)-phenoxy-phosphorylamino]propionate of formula **Ic** (3.99 mg, 8.26 mmol, 1 equiv.) in THF (16 ml) was added dropwise and the reaction mixture was stirred at -5°C. After 48 h, 2M HCl (100 ml) and subsequently EtOAc (100 ml) were added to the reaction mixture during 30 min. The organic layer was washed with 2M HCl (50 ml), 22 x 30 ml of 2.5 % Na₂CO₃, water (50 ml), brine (50 ml), and dried over Na₂SO₄. After removal of the solvent by distillation at a reduced pressure, 3.38 g of the crude product in the form of white foam was obtained. The crude product was dissolved in a DCM/toluene (20 ml) mixture at the room temperature, seeds of **II** were added and the mixture was agitated for 12 h. The product was aspirated on frit, washed with cold DCM and dried in a vacuum drier (40°C, 200 Pa) for 8 h, which provided 3.02 g (69%) of sofosbuvir of formula **II** in the form of white powder (purity 99.5%, optical purity >99.9%)
¹H NMR (500 MHz, DMSO) δ 11.50 (s, 1H), 7.53 (d, *J* = 7.0 Hz, 1H), 7.33-7.36 (m, 2H), 7.18-7.21 (m, 2H), 7.117-7.20 (m, 1H), 6.03 (dd, *J* = 12.7, 10.4 Hz, 1H), 5.83 (d, *J* = 4.3 Hz, 1H), 5.51 (dd, *J* = 8.1, 1.6 Hz, 1H), 5.51 (dd, *J* = 8.1, 1.6 Hz, 1H), 4.85 (hept, *J* = 6.3 Hz, 1H), 4.33 (dd, *J* = 11.1, 5.7 Hz, 1H), 4.20 (dt, *J* = 11.6, 6.0 Hz, 1H), 3.97 (dd, *J* = 9.1,4.7 Hz, 1H), 3.85 - 3.76 (m, 1H), 1.25 (d, *J* = 22.6 Hz, 1H), 1.22 (d, *J* = 6.8 Hz, 1H), 1.15 (dd, *J* = 6.3, 0.8 Hz, 1H). ¹³C NMR (126 MHz, DMSO) δ 172.64 (d, *J* = 5.1 Hz), 162.78 (s), 150.71 (d, *J* = 6.3 Hz), 150.47 (s), 129.67 (s), 124.63 (s), 120.09 (d, *J* = 4.9 Hz), 102.28 (s), 101.04 (s), 99.60 (s), 79.47 (s), 71.51 (s), 68.02 (s), 64.66 (s), 49.79 (s), 26.86 (s), 21.42 (d, *J* = 4.3 Hz), 19.81 (d, *J* = 6.5 Hz), 16.56 (d, *J* = 25.2 Hz).³¹P NMR (101 MHz, DMSO) δ 3.80 (s).HPLC (210 nm): *R*ₜ = 9.853 min.

### Example 16

### Sofosbuvir of formula II

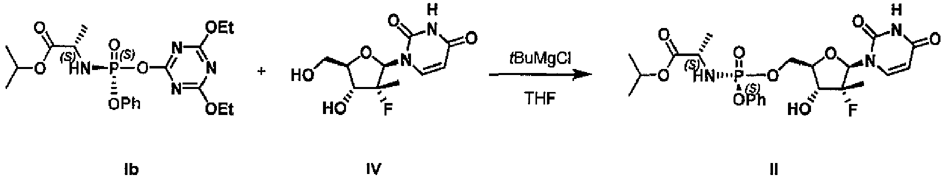

Using the procedure described in Example 15 with the use of isopropyl 2-(*S*)-[(*S*)-(4,6-diethoxy-1,3,5-triazin-2-oxy)-phenoxy-phosphorylamino]propionate of formula **Ib**, sofosbuvir of formula **II** was obtained in the yield of 63%.

### Example 17

### Sofosbuvir of formula II

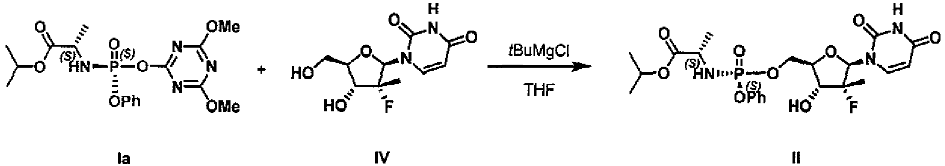

Using the procedure described in Example 15 with the use of isopropyl 2-(*S*)-[(*S*)-(4,6-dimethoxy-1,3,5-triazin-2-oxy)-phenoxy-phosphorylamino]propionate of formula la, sofosbuvir of formula **II** was obtained in the yield of 63%.

### Example 18

### Sofosbuvir of formula II

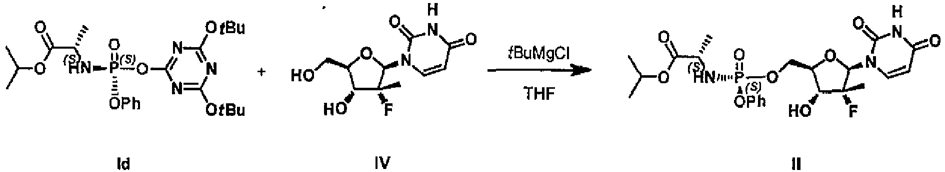

Using the procedure described in Example 15 with the use of isopropyl 2-(*S*)-[(*S*)-(4,6-di-*tert-*butyl-1,3,5-triazin-2-oxy)-phenoxy-phosphorylamino]propionate of formula **Id** sofosbuvir of formula **II** was obtained in the yield of 57%.

### Example 19

### Sofosbuvir of formula II

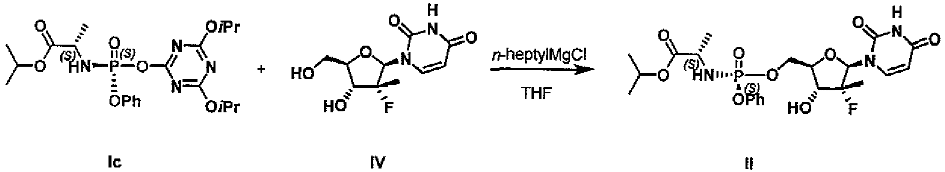

A solution of *n*-heptylMgCl (2.7 ml, 2.71 mmol, 2.05 equiv. 1M solution in THF) was added dropwise to a solution of the compound of formula **IV** (344 mg, 1.32 mmol) in THF (10 ml) at the temperature of -15°C during 1 h. The reaction mixture was agitated at -15°C for 1h, then heated up to -5°C during 30 min and agitated for another 2h. Then, a solution of 2-(*S*)-[(*S*)-(4,6-diisopropoxy-1,3,5-triazin-2-oxy)-phenoxy-phosphorylamino]propionate of formula **Ic** (606 mg, 1.26 mmol, 0.95 equiv.) in THF (2.6 ml) was added dropwise and the reaction mixture was stirred at -5°C. After 48 h, 2M HCl (15 ml) and subsequently EtOAc (15 ml) were added to the reaction mixture during 30 min. The organic layer was washed with 2M HCl (5 ml), 22 x 3 ml of 2.5 % Na₂CO₃, water (5 ml), brine (5 ml), and dried over Na₂SO₄. After removal of the solvent by distillation at a reduced pressure the amount of 489 mg of the crude product in the form of white foam was obtained. At the room temperature the crude product was dissolved in a DCM/toluene (5 ml) mixture, seeds of **II** were added and the mixture was agitated for 6 h. The product was aspirated on frit, washed with cold DCM and dried in a vacuum drier (40 °C, 200 Pa) for 4 h, which provided 426 g (61%) of sofosbuvir of formula **II** in the form of white powder (purity 99.1 %, optical purity >99.9 %).

## Claims

1. 1,3,5-Triazin-2-yl phosphoramidates of general formula **I,** 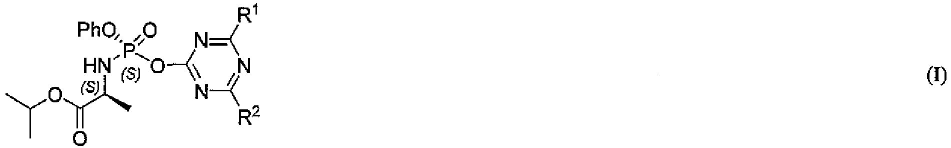 wherein R¹ and R² are independently H, a C1-C6 unbranched or branched alkyl, a C1-C6 unbranched or branched alkoxy group, a C1-C6 unbranched or branched alkylsulfanyl group, C1-C6 unbranched or branched monoalkylamino or dialkylamino group, pyrrolidino group, piperidino group, or morpholino group, as well as stereoisomers, salts, solvates, hydrates and crystalline and amorphous forms thereof.

2. The compounds in accordance with claim 1, **characterized in that** R¹ and R² are the same C1-C6 unbranched or branched alkoxy groups, preferably methoxy, ethoxy, propoxy, allyloxy, isopropoxy, or *tert*-butoxy groups.

3. The compound in accordance with claims 1 or 2, having the structure of formula **la,** 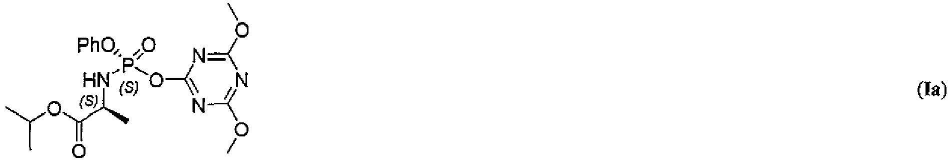 and whose name is isopropyl 2-(*S*)-[(*S*)-(4,6-dimethoxy-1,3,5-triazin-2-oxy)-phenoxy-phosphorylamino]-propionate.

4. The compound in accordance with claims 1 or 2, having the structure of formula **Ib,** 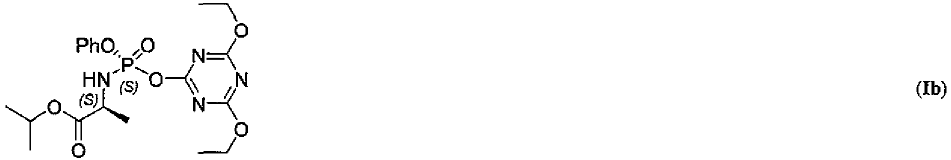 and whose name is isopropyl 2-(*S*)-[(*S*)-(4,6-diethoxy-1,3,5-triazin-2-oxy)-phenoxy-phosphorylamino] -propionate.

5. The compound in accordance with claims 1 or 2, having the structure of formula **Ic,** 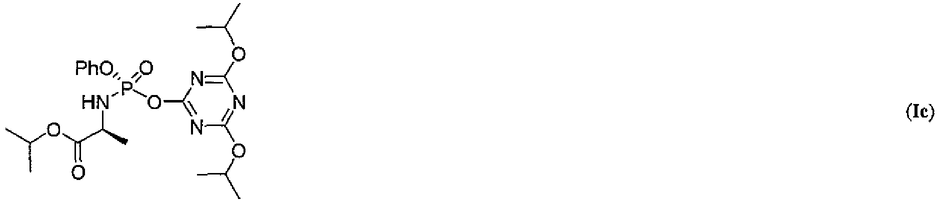 and whose name is isopropyl 2-(*S*)-[(*S*)-(4,6-diisopropoxy-1,3,5-triazin-2-oxy)-phenoxy-phosphorylamino]-propionate.

6. The compound in accordance with claims 1 or 2, having the structure of formula **Id,** 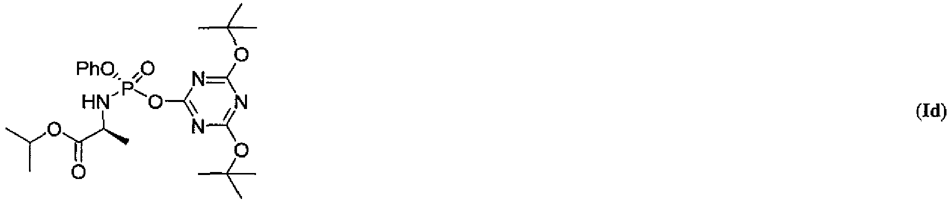 and whose name is isopropyl 2-(*S*)-[(*S*)-(4,6-di-tert-butoxy-1,3,5-triazin-2-oxy)-phenoxy-phosphorylamino]-propionate.

7. A process for the production of compounds of formula **I** 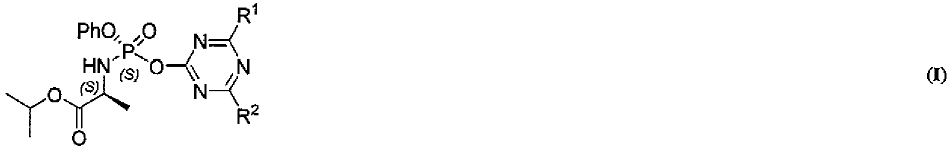 as defined in claims 1 to 6, comprising a reaction of the chloro derivative of formula **V** with triazine derivatives of formula **VI,** 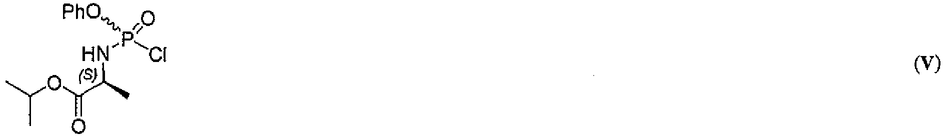 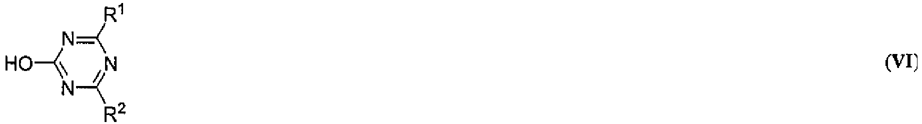 in the presence of a suitable base in suitable solvents, **characterized in that** the individual isomers as **characterized in** claims 1 and 6 are obtained from the resulting mixture of the diastereoisomers of the compound of formula **IX** 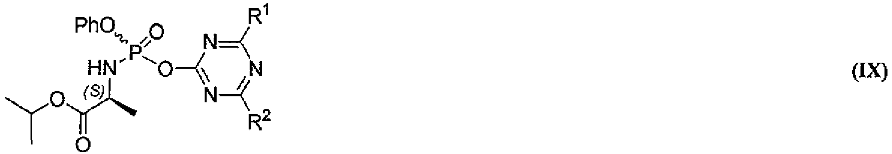 either by direct separation and/or by crystallization-induced dynamic resolution and subsequent separation, wherein the starting compound **V** is generated *in situ* by a reaction of (*S*)-isopropyl 2-aminopropanoate (L-alanine isopropyl ester) with phenyl dichlorophosphate.

8. The process in accordance with claim 7, **characterized in that** said suitable base is selected from the group including triethylamine, N,N-diisopropylethylamine, 1,8-diazabicyclo[5.4.0.]undec-7-ene, 1,4-diazabicyclo[2.2.2]octane, 1-methyl morpholine, 1-ethylpiperidine, Na₂CO₃, K₂CO₃, Cs₂CO₃, NaHCO₃, KHCO₃, NaOAc and KOAc, preferably triethylamine or N,N-diisopropylethylamine.

9. The process in accordance with claims 7 or 8, **characterized in that** said suitable solvent is selected from the group including dichloromethane, chloroform, tetrahydrofuran, 2-methyltetrahydrofuran, cyclopentyl methyl ether, dimethoxyethane, methyl *tert-butyl* ether, toluene, trifluorotoluene; preferably dichloromethane, tetrahydrofuran, 2-methyltetrahydrofuran, cyclopentyl methyl ether.

10. The process in accordance with claims 7 to 9, **characterized in that** from the resulting mixture of the diastereoisomers of the compound of formula **I** the Sp diastereoisomers are separated in the form of crystals with the chiral purity of 99% and higher.

11. The process in accordance with claims 7 to 9, **characterized in that** the mother liquors, obtained after the separation of chirally very pure crystals of the Sp diastereoisomers of the compound of formula **I,** are subjected to crystallization-induced dynamic resolution, and another fraction of crystals of the Sp diastereoisomers of the compound of formula **I** with the chiral purity of 99% and higher is obtained.

12. The process in accordance with claim 7, **characterized in that** the Sp diastereoisomers of the compound of formula **I** are obtained by crystallization-induced dynamic resolution, wherein a single fraction of the Sp diastereoisomers of the compound of formula **I** with the chiral purity of 99% and higher is obtained.

13. The process in accordance with claims 11 and 12, **characterized in that** said crystallization-induced dynamic resolution is conducted by treating mixtures of the diastereoisomers with a suitable base in a suitable solvent.

14. The process in accordance with claim 13, **characterized in that** said base is triethylamine, N,N-diisopropylethylamine, 1,8-diazabicyclo[5.4.0.]undec-7-ene, 1,4-diazabicyclo[2.2.2]octane, imidiazole, 1,2-bis(dimethylamino)ethane, 1,2-bis(diethylamino)ethane, 1,3-bis(dimethylamino)propane, or 1,3-bis(diethylamino)-propane; preferably triethylamine or N,N-diisopropylethylamine.

15. The process in accordance with claims 13 and 14, **characterized in that** said solvent is ethyl acetate, isopropyl acetate, 2-methyltetrahydrofuran, tetrahydrofuran, dioxane, dimethoxyethane, diethoxyethane, *tert-butyl* methyl ether, cyclopentyl methyl ether, diethyl ether, toluene, methyl ethyl ketone, acetone, pentane, hexane, heptane, cyclohexane, decalin, methylcyclohexane, 2-methylbutane, 2-methylpentane, triethylmethane, isooctane, toluene, xylene, benzene, trifluorotoluene or mixtures thereof; preferably ethyl acetate, isopropyl acetate, 2-methyltetrahydrofuran, tetrahydrofuran, hexane, heptane, cyclohexane or mixtures thereof.

16. A process for the production of sofosbuvir of formula **II,** 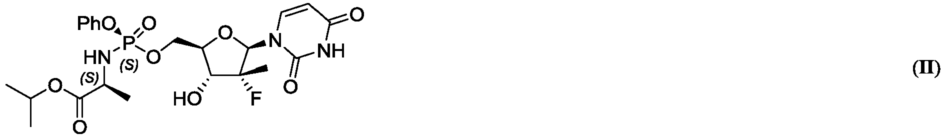 **characterized in that** it comprises a reaction of the intermediate **IV** 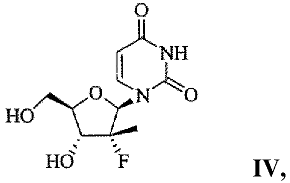 activated with a suitable agent, with the compounds of formula **I** as defined in claims 1 to 6 in a suitable solvent at a suitable temperature.

17. The process for the production of sofosbuvir of formula **II** in accordance with claim 16, **characterized in that** a Grignard reagent is used as the activation agent.

18. The process in accordance with claims 16 and 17, **characterized in that** said Grignard reagent is *tert*-butyl magnesium chloride, benzylmagnesium chloride, cyclohexylmagnesium chloride, cyclohexylmagnesium bromide, heptylmagnesium chloride, or octylmagnesium chloride; preferably heptylmagnesium chloride or *tert-*butyl magnesium chloride.

19. The process in accordance with claims 16 to 18, **characterized in that** the Grignard reagent : intermediate **IV** are used in the ratios of 1.1:1 to 2.9:1 molar equivalents; preferably in the ratios of 1.8:1 to 2.2:1.

20. The process in accordance with claims 16 to 19, **characterized in that** said solvent is tetrahydrofuran, 2-methyltetrahydrofuran, dioxane, *tert*-butyl methyl ether, cyclopentyl methyl ether, diethyl ether, dimethoxyethane, diethoxyethane, dimethoxypropane, or diethoxypropane; preferably tetrahydrofuran, 2-methyltetrahydrofuran or cyclopentyl methyl ether.

21. The process in accordance with claims 16 to 20, **characterized in that** said activated intermediate **IV** is reacted with the compounds of formula **I** at the temperatures of -20 to 20°C, preferably at the temperatures of -10 to 10°C.

22. The process in accordance with claims 16 to 21, **characterized in that** said activated intermediate IV is reacted with the compounds of formula I in the molar ratios of 10:1 to 1:10, preferably 2:1 to 1:2.

## Patentansprüche

1. 1,3,5-Triazin-2-yl-phosphoramidate der allgemeinen Formel I, 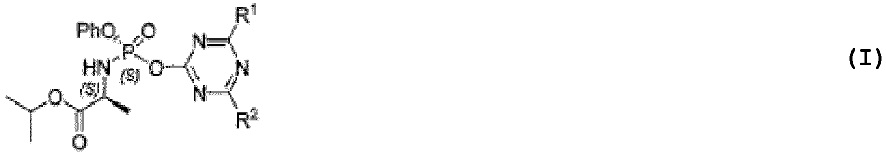 worin R¹ und R² unabhängig voneinander für H, ein unverzweigtes oder verzweigtes C1-C6-Alkyl, eine unverzweigte oder verzweigte C1-C6-Alkoxygruppe, eine unverzweigte oder verzweigte C1-C6-Alkylsulfanylgruppe, eine unverzweigte oder verzweigte C1-C6-Monoalkylamino- oder Dialkylaminogruppe, Pyrrolidinogruppe, Piperidinogruppe oder Morpholinogruppe, sowie für Stereoisomere, Salze, Solvate, Hydrate und kristalline und amorphe Formen davon, stehen.

2. Verbindungen nach Anspruch 1, **dadurch gekennzeichnet, dass** R¹ und R² für die gleichen unverzweigten oder verzweigten C1-C6-Alkoxygruppen, vorzugsweise Methoxy-, Ethoxy-, Propoxy-, Allyloxy-, Isopropoxy- oder *tert*-Butoxygruppen, stehen.

3. Verbindung nach Anspruch 1 oder 2 mit der Struktur der Formel Ia, 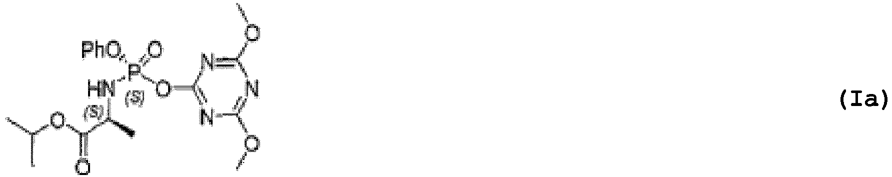 wobei der Name der Verbindung Isopropyl-2-(*S*)-[(*S*)-(4,6-dimethoxy-1,3,5-triazin-2-oxy)phenoxy-phosphorylamino]-propionat ist.

4. Verbindung nach den Ansprüchen 1 oder 2 mit der Struktur der Formel Ib, 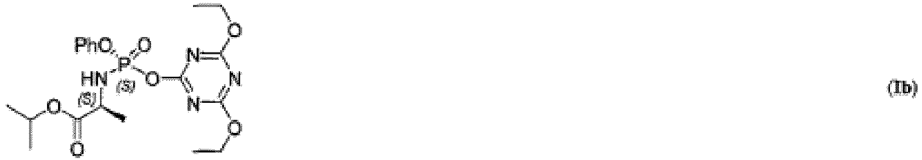 wobei der Name der Verbindung Isopropyl-2-(*S*)-[(*S*)-(4,6-diethoxy-1,3,5-triazin-2-oxy)phenoxy-phosphorylamino]-propionat ist.

5. Verbindung nach den Ansprüchen 1 oder 2 mit der Struktur der Formel Ic, 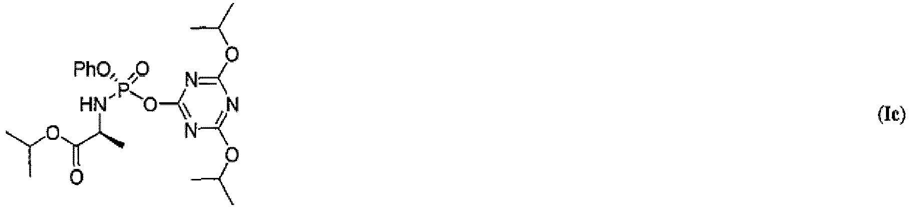 wobei der Name der Verbindung Isopropyl-2-(*S*)-[(*S*)-(4,6-diisopropoxy-1,3,5-triazin-2-oxy)phenoxy-phosphoryl-amino]propionat ist.

6. Verbindung nach Anspruch 1 oder 2 mit der Struktur der Formel Id, 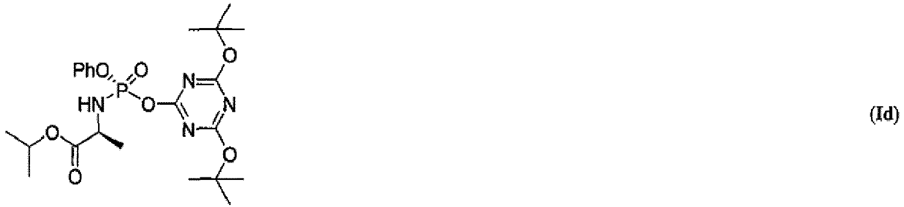 wobei der Name der Verbindung Isopropyl-2-(*S*)-[(*S*)-(4,6-di-tert-butoxy-1,3,5-triazin-2-oxy)phenoxy-phosphorylamino]-propionat ist.

7. Verfahren zur Herstellung der Verbindungen der Formel I 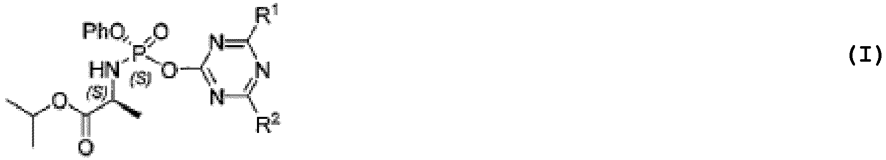 nach den Ansprüchen 1 bis 6, umfassend die Umsetzung des Chlorderivats der Formel V mit Triazinderivaten der Formel VI 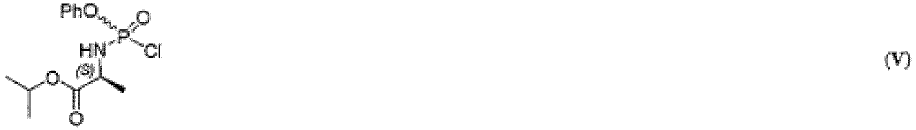 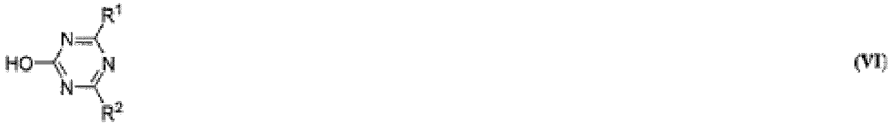 in Gegenwart einer geeigneten Base in geeigneten Lösungsmitteln, **dadurch gekennzeichnet, dass** die einzelnen Isomeren, wie sie in den Ansprüchen 1 und 6 gekennzeichnet sind, aus der resultierenden Mischung der Diastereoisomeren der Verbindung der Formel IX 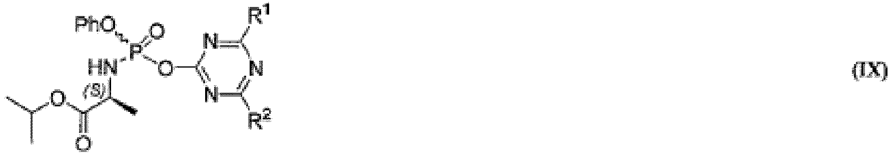 entweder durch direkte Trennung und/oder durch kristallisationsinduzierte dynamische Spaltung und anschließende Trennung, wobei die Ausgangsverbindung V in situ durch eine Reaktion von (*S*)-Isopropyl-2-aminopropanoat (L-Alaninisopropylester) mit Phenyldichlorphosphat hergestellt wird, erhalten werden.

8. Verfahren nach Anspruch 7, **dadurch gekennzeichnet, dass** die geeignete Base ausgewählt ist aus der Gruppe, die Triethylamin, N,N-Diisopropylethylamin, 1,8-Diazabicyclo[5.4.0]undec-7-en, 1,4-Diazabicyclo[2.2.2]octan, 1-Methylmorpholin, 1-Ethylpiperidin, Na₂CO₃, K₂CO₃, Cs₂CO₃, NaHCO₃, KHCO₃, NaOAc und KOAc, vorzugsweise Triethylamin oder N,N-Diisopropylethylamin umfasst.

9. Verfahren nach Anspruch 7 oder 8, **dadurch gekennzeichnet, dass** das geeignete Lösungsmittel aus der Gruppe ausgewählt ist, die Dichlormethan, Chloroform, Tetrahydrofuran, 2-Methyltetrahydrofuran, Cyclopentylmethylether, Dimethoxyethan, Methyl-*tert*-butylether, Toluol, Trifluortoluol, vorzugsweise Dichlormethan, Tetrahydrofuran, 2-Methyltetrahydrofuran, Cyclopentylmethylether, umfasst.

10. Verfahren nach den Ansprüchen 7 bis 9, **dadurch gekennzeichnet, dass** aus der resultierenden Mischung der Diastereoisomeren der Verbindung der Formel I die Sp-Diastereoisomere in Form von Kristallen mit einer chiralen Reinheit von 99 % und höher abgetrennt werden.

11. Verfahren nach den Ansprüchen 7 bis 9, **dadurch gekennzeichnet, dass** die Mutterlaugen, die nach der Trennung von chiral sehr reinen Kristallen der Sp-Diastereoisomere der Verbindung der Formel I erhalten werden, einer kristallisationsinduzierten dynamischen Spaltung unterworfen werden und eine weitere Fraktion von Kristallen der Sp-Diastereoisomere der Verbindung der Formel I mit der chiralen Reinheit von 99 % und höher erhalten wird.

12. Verfahren nach Anspruch 7, **dadurch gekennzeichnet, dass** die Sp-Diastereoisomere der Verbindung der Formel I durch kristallisationsinduzierte dynamische Spaltung erhalten werden, wobei eine einzelne Fraktion der Sp-Diastereoisomere der Verbindung der Formel I mit der chiralen Reinheit von 99 % und höher erhalten wird.

13. Verfahren nach den Ansprüchen 11 und 12, **dadurch gekennzeichnet, dass** die kristallisationsinduzierte dynamische Trennung durch Behandlung von Mischungen der Diastereoisomeren mit einer geeigneten Base in einem geeigneten Lösungsmittel durchgeführt wird.

14. Verfahren nach Anspruch 13, **dadurch gekennzeichnet, dass** die Base Triethylamin, N,N-Diisopropylethylamin, 1,8-Diazabicyclo[5.4.0]undec-7-en, 1,4-Diazabicyclo[2.2.2]-octan, Imidiazol, 1,2-Bis(dimethylamino)ethan, 1,2-Bis-(diethylamino)ethan, 1,3-Bis(dimethylamino)propan oder 1,3-Bis(diethylamino)propan, vorzugsweise Triethylamin oder N,N-Diisopropylethylamin ist.

15. Verfahren nach den Ansprüchen 13 und 14, **dadurch gekennzeichnet, dass** das Lösungsmittel Ethylacetat, Isopropylacetat, 2-Methyltetrahydrofuran, Tetrahydrofuran, Dioxan, Dimethoxyethan, Diethoxyethan, tert-Butylmethylether, Cyclopentylmethylether, Diethylether, Toluol, Methylethylketon, Aceton, Pentan, Hexan, Heptan, Cyclohexan, Decalin, Methylcyclohexan, 2-Methylbutan, 2-Methylpentan, Triethylmethan, Isooctan, Toluol, Xylol, Benzol, Trifluortoluol oder Mischungen davon ist, vorzugsweise Ethylacetat, Isopropylacetat, 2-Methyltetrahydrofuran, Tetrahydrofuran, Hexan, Heptan, Cyclohexan oder Mischungen davon.

16. Verfahren zur Herstellung von Sofosbuvir der Formel II 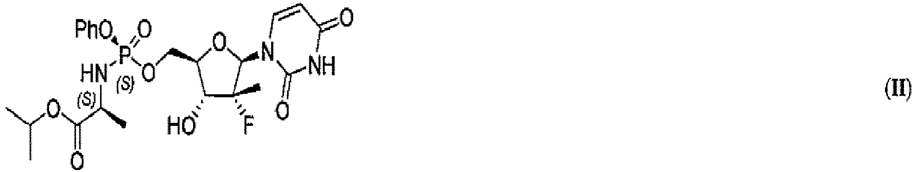 **dadurch gekennzeichnet, dass** es eine Reaktion des Zwischenprodukts IV 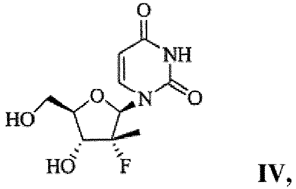 aktiviert mit einem geeigneten Mittel, mit den in den Ansprüchen 1 bis 6 definierten Verbindungen der Formel I in einem geeigneten Lösungsmittel, bei einer geeigneten Temperatur umfasst.

17. Verfahren zur Herstellung von Sofosbuvir der Formel II nach Anspruch 16, **dadurch gekennzeichnet, dass** als Aktivierungsmittel ein Grignard-Reagenz verwendet wird.

18. Verfahren nach den Ansprüchen 16 und 17, **dadurch gekennzeichnet, dass** das Grignard-Reagenz tert-Butylmagnesiumchlorid, Benzylmagnesiumchlorid, Cyclohexylmagnesiumchlorid, Cyclohexylmagnesiumbromid, Heptylmagnesiumchlorid oder Octylmagnesiumchlorid, vorzugsweise Heptylmagnesiumchlorid oder tert-Butylmagnesiumchlorid, ist.

19. Verfahren nach den Ansprüchen 16 bis 18, **dadurch gekennzeichnet, dass** das Grignard-Reagenz : Zwischenprodukt IV in Verhältnissen von 1,1:1 bis 2,9:1 Moläquivalenten eingesetzt wird, vorzugsweise in Verhältnissen von 1,8:1 bis 2,2:1.

20. Verfahren nach den Ansprüchen 16 bis 19, **dadurch gekennzeichnet, dass** das Lösungsmittel Tetrahydrofuran, 2-Methyltetrahydrofuran, Dioxan, tert-Butylmethylether, Cyclopentylmethylether, Diethylether, Dimethoxyethan, Diethoxyethan, Dimethoxypropan oder Diethoxypropan ist, vorzugsweise Tetrahydrofuran, 2-Methyltetrahydrofuran oder Cyclopentylmethylether.

21. Verfahren nach den Ansprüchen 16 bis 20, **dadurch gekennzeichnet, dass** das aktivierte Zwischenprodukt IV mit den Verbindungen der Formel I bei Temperaturen von -20 bis 20 °C, vorzugsweise bei Temperaturen von -10 bis 10 °C umgesetzt wird.

22. Verfahren nach den Ansprüchen 16 bis 21, **dadurch gekennzeichnet, dass** das aktivierte Zwischenprodukt IV mit den Verbindungen der Formel I in Molverhältnissen von 10:1 bis 1:10, vorzugsweise 2:1 bis 1:2, umgesetzt wird.

## Revendications

1. 1,3,5-triazin-2-yl-hoshoramidates de formule générale I, 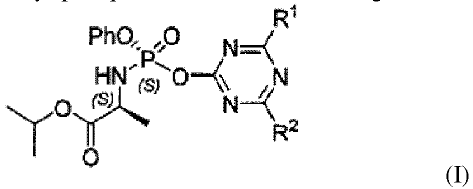 dans laquelle R¹ et R² sont, indépendamment l'un de l'autre, l'atome d'hydrogène, un groupe alkyle en C1-C6 non ramifié ou ramifié, un groupe alcoxy en C1-C6 non ramifié ou ramifié, un groupe alkylsulfanyle en C1-C6 non ramifié ou ramifié, un groupe monoalkylamino ou dialkylamino en C1-C6 non ramifié ou ramifié, un groupe pyrrolidine, un groupe pipéridino ou un groupe morpholino, ainsi que leurs stéréoisomères, sels, solvates, hydrates et leurs formes cristallines et amorphes.

2. Les composés selon la revendication 1, **caractérisés en ce que** R¹ et R² sont les mêmes groupes alcoxy en C1-C6 non ramifiés ou ramifiés, de préférence les groupes méthoxy, éthoxy, propoxy, allyloxy, isopropoxy ou tert-butoxy.

3. Le composé selon la revendication 1 ou 2, ayant la structure de la formule la, 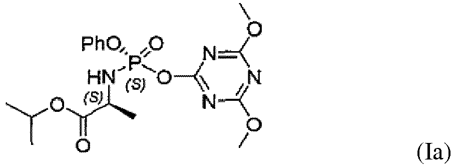 et dont le nom est 2-(S)-[(S)-(4,6-diméthoxy-1,3,5-triazin-2-oxy)-phénoxy-phosphorylamino]-propionate d'isopropyle.

4. Le composé selon les revendications 1 ou 2, ayant la structure de la formule 1b, 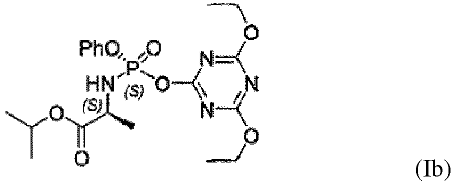 et dont le nom est 2-(S)-[(S)-(4,6-diéthoxy-1,3,5-triazin-2-oxy)-phénoxy-phosphorylamino]-propionate d'isopropyle.

5. Le composé selon les revendications 1 ou 2, ayant la structure de la formule Ic, 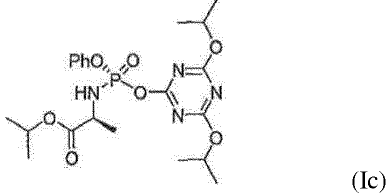 et dont le nom est 2-(S)-[(S)-(4,6-diisopropoxy-1,3,5-triazin-2-oxy)-phénoxy-phosphorylamino]_propionate d'isopropyle.

6. Le composé selon la revendication 1 ou 2, ayant la structure de la formule Id, 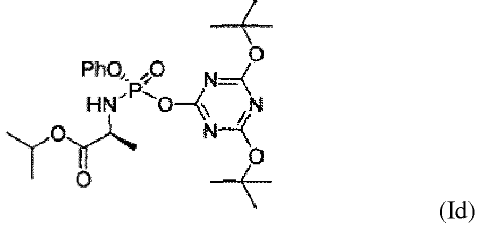 et dont le nom est le 2-(S)-[(S)-(4,6-di-tert-butoxy-1,3,5-triazin-2-oxy)-phénoxy-phosphorylamino]-propionate d'isopropyle.

7. Le procédé de préparation des composés de formule I 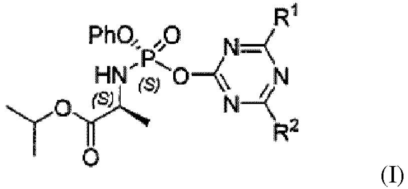 tels que définis dans les revendications 1 à 6, comprenant une réaction du dérivé chloré de formule V avec des dérivés de triazine de formule VI, 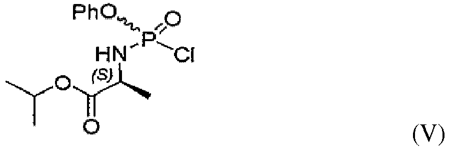 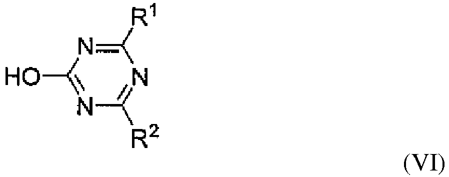 en présence d'une base appropriée dans des solvants appropriés, **caractérisée en ce que** les isomères individuels tels que caractérisés dans les revendications 1 et 6 sont obtenus à partir du mélange résultant des diastéréoisomères du composé de formule IX, 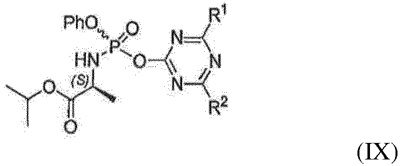 soit par séparation directe et/ou par résolution dynamique induite par cristallisation et séparation subséquente, dans lequel procédé le composé de départ V est généré *in situ* par réaction du (S)-isopropyl-2-aminopropanoate(L-alanine isopropyl ester) avec le phényl dichlorophosphate.

8. Le procédé selon la revendication 7, **caractérisé en ce que** ladite base appropriée est choisie dans le groupe comprenant la triéthylamine, la N, N-diisopropyléthylamine, le 1,8-diazabicyclo[5.4.0.]undéc-7-ène, 1,4-diazabicyclo. [2.2.2]octane, 1-méthylmorpholine, 1-éthylpipéridine, Na₂CO₃, K₂CO₃, Cs₂CO₃, NaHCO₃, KHCO₃, NaOAc et KOAc, de préférence la triéthylamine ou la N, N-diisopropyléthylamine.

9. Le procédé selon les revendications 7 ou 8, **caractérisé en ce que** ledit solvant approprié est choisi dans le groupe comprenant le dichlorométhane, le chloroforme, le tétrahydrofuranne, le 2-méthyltétrahydrofurane, le cyclopentylméthyléther, le diméthoxyéthane, le méthyl-tert-butyléther, le toluène, le trifluorotoluène; de préférence le dichlorométhane, le tétrahydrofuranne, le 2-méthyltétrahydrofurane, le cyclopentylméthyléther.

10. Le procédé selon les revendications 7 à 9, **caractérisé en ce qu'**à partir du mélange résultant des diastéréoisomères du composé de formule I, les diastéréoisomères Sp sont séparés sous la forme de cristaux présentant une pureté chirale de 99% et plus.

11. Le procédé selon les revendications 7 à 9, **caractérisé en ce que** les liqueurs mères, obtenues après la séparation des cristaux chiralement très purs des diastéréoisomères Sp du composé de formule I, sont soumises à une résolution dynamique induite par cristallisation, et est obtenue une autre fraction des cristaux des diastéréoisomères Sp du composé de formule I avec une pureté chirale de 99% et plus.

12. Le procédé selon la revendication 7, **caractérisé en ce que** les diastéréoisomères Sp du composé de formule I sont obtenus par résolution dynamique induite par cristallisation, dans lequel une fraction unique des diastéréoisomères Sp du composé de formule I ayant une pureté chirale de 99% et plus élevée est obtenue.

13. Le procédé selon les revendications 11 et 12, **caractérisé en ce que** ladite résolution dynamique induite par cristallisation est effectuée par traitement des mélanges de diastéréoisomères avec une base appropriée dans un solvant approprié.

14. Le procédé selon la revendication 13, **caractérisé en ce que** ladite base est la triéthylamine, la N, N-diisopropyléthylamine, le 1,8-diazabicyclo [5.4.0.] undéc-7-ène, le 1,4-diazabicyclo [2.2.2] octane, l'imidiazole, le 1,2-bis (diméthylamino) éthane, le 1,2-bis (diéthylamino) éthane, le 1,3-bis (diméthylamino) propane ou le 1,3-bis (diéthylamino)-propane; de préférence la triéthylamine ou la N,N-diisopropyléthylamine.

15. Le procédé selon les revendications 13 et 14, **caractérisé en ce que** ledit solvant est l'acétate d'éthyle, l'acétate d'isopropyle, le 2-méthyl-tetrahydrofuranne, le tétrahydrofuranne, le dioxanne, le diméthoxyéthane, le diéthoxyéthane, le tert-butylméthyl éther, le cyclopentylméthyl éther, le diéthyléther, le toluène, la méthyléthyl cétone, l'acétone, le pentane, l'hexane, l'heptane, le cyclohexane, la décaline, le méthylcyclohexane, le 2-méthylbutane, le 2-méthylpentane, le triéthylméthane, l'isooctane, le toluène, le xylène, le benzène, le trifluorotoluène ou leurs mélanges; de préférence l'acétate d'éthyle, l'acétate d'isopropyle, le 2-méthyltétrahydrofurane, le tétrahydrofuranne, l'hexane, l'heptane, le cyclohexane ou leurs mélanges.

16. Un procédé de production de sofosbuvir de formule II 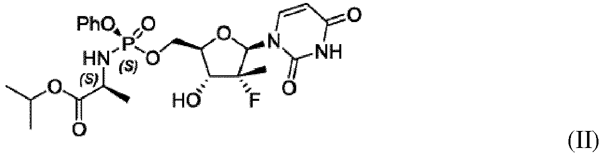 **caractérisé en ce qu'**il comprend une réaction de l'intermédiaire IV, 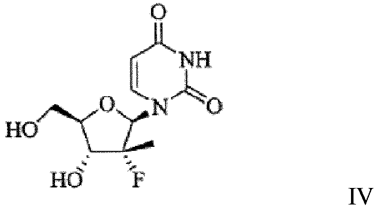 activé avec un agent approprié, avec les composés de formule I tels que définis dans les revendications 1 à 6 dans un solvant à une température appropriée.

17. Le procédé de production de sofosbuvir de formule II selon la revendication 16, **caractérisé en ce que** l'on utilise comme agent d'activation un réactif de Grignard.

18. Le procédé selon les revendications 16 et 17, **caractérisé en ce que** ledit réactif de Grignard est le chlorure de tert-butylmagnésium, le chlorure de benzylmagnésium, le chlorure de cyclohexylmagnésium, le bromure de cyclohexylmagnésium, le chlorure d'heptylmagnésium ou le chlorure d'octylmagnésium; de préférence le chlorure d'heptylmagnésium ou le chlorure de tert-butylmagnésium.

19. Le procédé selon les revendications 16 à 18, **caractérisé en ce que** les réactifs de Grignard/intermédiaire IV sont utilisés dans les proportions en équivalents molaires de 1,1/1 à 2,9/1; de préférence dans les rapports de 1,8/1 à 2,2/1.

20. Le procédé selon les revendications 16 à 19, **caractérisé en ce que** ledit solvant est le tétrahydrofurane, le 2-méthyltétrahydrofurane, le dioxane, le tert-butylméthyléther, le cyclopentylméthyléther, le diéthyléther, le diméthoxyéthane, le diéthoxyéthane, le diméthoxypropane ou le diéthoxypropane; de préférence le tétrahydrofurane, le 2-méthyltétrahydrofurane ou le cyclopentylméthyléther.

21. Le procédé selon les revendications 16 à 20, **caractérisé en ce que** l'on fait réagir ledit intermédiaire activé IV avec les composés de formule I aux températures de -20 à 20°C, de préférence aux températures de -10 à 10°C.

22. Le procédé selon les revendications 16 à 21, **caractérisé en ce que** ledit intermédiaire IV activé réagit avec les composés de formule I dans les rapports molaires de 10/1 à 1/10, de préférence de 2/1 à 1/2.
